# EUROPEAN PATENT SPECIFICATION

(11) **EP 4 255 386 B1**
(45) Date of publication and mention of the grant of the patent: **26.03.2025**
(21) Application number: 21814804.7
(22) Date of filing: 24.11.2021
(51) Int. Cl.: A61K 8/81, A61Q 15/00

(54) **ANTIPERSPIRANT COMPOSITIONS**
SCHWEISSHEMMENDE ZUSAMMENSETZUNGEN
COMPOSITIONS ANTITRANSPIRANTES

(30) Priority: 07.12.2020 EP 20212291
(43) Date of publication of application: 11.10.2023
(73) Proprietor: Unilever IP Holdings B.V., 3013 AL Rotterdam (NL); Unilever Global IP Limited, Wirral, Merseyside CH62 4ZD (GB)
(72) Inventor: FIDGE, Christopher, Wirral, Merseyside CH63 3JW (GB); SKINNER, Richard, Wirral, Merseyside CH63 3JW (GB); TAYLOR, Cheryl, Anne, Wirral, Merseyside CH63 3JW (GB)
(74) Representative: Whaley, Christopher
(86) International application number: PCT/EP2021/082808
(87) International publication number: WO 2022/122385

(56) References cited:
- WO-A2-2008/036587
- WO-A2-2012/175330
- US-A- 4 060 678
- US-A1- 2007 248 551
- US-A1- 2014 242 015
- US-A1- 2017 189 290
- US-B2- 10 292 915
- US-B2- 7 892 525
- DATABASE GNPD [online] MINTEL; 4 August 2010 (2010-08-04), ANONYMOUS: "Control Cream", XP055893024, retrieved from https://www.gnpd.com/sinatra/recordpage/1379879/ Database accession no. 1379879
- DATABASE GNPD [online] MINTEL; 31 May 2005 (2005-05-31), ANONYMOUS: "Lifting Action Day Cream", XP055893018, retrieved from https://www.gnpd.com/sinatra/recordpage/364052/ Database accession no. 364052
- DATABASE GNPD [online] MINTEL; 4 August 2010 (2010-08-04), ANONYMOUS: "Control Cream", XP055893024, retrieved from https://www.gnpd.com/sinatra/recordpage/1379879/ Database accession no. 1379879
- DATABASE GNPD [online] MINTEL; 31 May 2005 (2005-05-31), ANONYMOUS: "Lifting Action Day Cream", XP055893018, retrieved from https://www.gnpd.com/sinatra/recordpage/364052/ Database accession no. 364052

## Description

### Field of Invention

The present invention is in the field of antiperspirant compositions and methods of achieving reduced perspiration.

### Background

There have been numerous references to the use of 2-dimethylaminoethyl methacrylate (DMAEMA) polymers in cosmetic compositions, but relatively few to their use in antiperspirant compositions.

WO 2012/175330 A (L'Oreal, 2012) discloses the cosmetic use of flocculant polymer as antiperspirant actives, including polymers comprising dimethylaminoethyl (meth)acrylate monomer units.

WO 2010/070140 A (L'Oreal, 2010) discloses antiperspirant compositions comprising a complex of an anionic species and a cationic species, wherein the cationic species may be a dialkylaminoalkyl acrylate or methacrylate.

### Summary of Invention

It is an object of the invention to reduce perspiration on the surface of the human body, in particular by the use of selected antiperspirant agents that do not comprise aluminium or zirconium or any other metal salts.

It is a further object of the invention to reduce perspiration on the surface of the human body by the use an antiperspirant composition comprising an organic antiperspirant active.

The present invention aims to provide the non-therapeutic cosmetic use of poly(DMAEMA) as an antiperspirant agent, wherein the poly(DMAEMA) is a homopolymer.

In a second aspect of the invention, there is provided a cosmetic method of reducing perspiration comprising the topical application of a composition comprising poly(DMAEMA) as an antiperspirant agent and a cosmetically acceptable carrier fluid.

In a third aspect of the invention, there is provided an antiperspirant composition comprising poly(DMAEMA), as defined in the first aspect of the invention; a cosmetically acceptable carrier fluid; and a preservative and/or deodorant active.

In a fourth aspect of the invention, there is provided an antiperspirant product comprising a composition as described in the third aspect of the invention and a dispenser for said composition, the dispenser comprising containment means and application means for the composition.

In the cosmetic method described in the second aspect of the invention, the composition preferably comprises a cosmetically acceptable carrier fluid and a preservative and/or deodorant active.

### Detailed Description

Herein, poly(DMAEMA) is an abbreviation of poly(2-dimethylaminoethyl methacylate) and should be understood to mean homopolymer.

In preferred embodiments, the poly(DMAEMA) comprises at least five consecutive linked DMAEMA residues

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, all percentages, ratios and amounts are by weight, unless otherwise indicated.

Herein, all percentages, ratios and amounts are to be understood to be modified by the word "about" where appropriate.

Herein, the word "comprising" is intended to mean "including" but not necessarily "consisting of", i.e., it is non-exhaustive.

Herein, cosmetic methods are to be understood as a non-therapeutic in nature and *vice versa.*

Herein, references to an amount of a compound or an active refer to the total amount of compounds or actives of the type indicated.

Herein, "application" and "applied" relate to application to the surface of the human body, in particular the underarm regions, unless the context dictates otherwise.

Herein, molecular weights for poly(DMAEMA) refer to number average molecular weights, unless otherwise indicated.

The invention typically involves the topical application of the antiperspirant active to the underarm regions of the human body, otherwise known as the axillae.

Poly(DMAEMA) is an essential feature of the present invention. The DMAEMA residues in the poly(DMAEMA) may be neutral, protonated (i.e. cationic) or quaternized (i.e. also cationic). The DMAEMA residues may be made cationic by the addition of an acid, such as hydrochloric acid, leading to poly(DMAEMA.hydrochloride), for example. The DMAEMA residues may be quaternized by the addition of methyl chloride, for example, leading to poly(DMAEMA, methyl chloride quaternary salt), for example. Both protonated and quaternized poly(DMAEMA) are poly(DMAEMA) according to the present invention. In some preferred embodiments, the poly(DMAEMA) is cationic.

In some embodiments, the molecular weight of the poly(DMAEMA) is preferably at least 1000 Da, for example from 1000 Da to 1,000,000 Da. In other embodiments, particularly those in which the poly(DMAEMA) is dissolved in a cosmetically acceptable carrier fluid, the molecular weight of the poly(DMAEMA) is preferably from 250,000 to 750,000 Da and more preferably from 300,000 to 450,000 Da.

The poly(DMAEMA) antiperspirant active is preferably used from a composition comprising it at a level of from 0.1 to 30%, preferably from 0.5 to 20% and more preferably from 1 to 15%.

In preferred embodiments, the poly(DMAEMA) is delivered to the skin surface at a concentration of at least 0.05% by weight and more preferably at a concentration of from 0.05 to 1.0% by weight.

Compositions according to the invention comprise a carrier fluid that aids the delivery of the poly(DMAEMA) to where it is required on the skin surface, which is typically in the vicinity of the sweat pores, particularly in the underarm areas of the human body.

The carrier fluid used in conjunction with the present invention must be cosmetically acceptable and it generally aids in the delivery of the non-pore blocking antiperspirant agent to the surface of the human body. The non-pore blocking antiperspirant agent is typically suspended or dissolved in the carrier fluid.

The carrier fluid may comprise from 10 to 99% of the composition, excluding any volatile propellant that may be present therein.

In preferred aspects of the present invention, the carrier fluid for the non-pore blocking antiperspirant agent comprises a C₂-C₆ vicinal diol.

Herein, C₂-C₆ vicinal diols are compounds with from 2 to 6 carbon atoms in which two neighbouring carbon atoms bear hydroxyl groups.

Examples of C₂-C₆ vicinal diols include ethylene glycol, propylene glycol, glycerol and hexane-1,2-diol.

Preferred C₂-C₆ vicinal diols exclude compounds comprising more than 3 hydroxyl groups.

Preferred C₂-C₆ vicinal diols are C₂-C₄ vicinal diol, i.e. compounds with from 2 to 4 carbon atoms in which two neighbouring carbon atoms bear hydroxyl groups.

When employed, the C₂-C₆ vicinal diol in compositions of the invention is preferably present at from 1 to 50%, more preferably from 2 to 40% and most preferably from 5 to 25%.

A preferred additional component of the carrier fluid is ethanol. This may comprise up to 80% of the total composition, but is typically restricted to 70%, and often less than 60%. When employed ethanol typically comprises at least 10%, preferably at least 20%, and more preferably at least 40% by weight of total composition. Each of these preferred minimum levels of ethanol may be limited by the aforementioned maximum levels of ethanol.

A preferred component of the carrier fluid is water, especially when employed in conjunction with ethanol, to give an aqueous ethanol carrier fluid. Water may comprise up to 90% of the total composition, but is typically restricted to less than 60%, and often less than 50%. When employed water typically comprises at least 10%, preferably at least 20%, and more preferably at least 30% by weight of total composition. Each of these preferred minimum levels of water may be limited by the aforementioned maximum levels of water.

A preservative or deodorant active is at least a preferred feature in each aspect of the invention.

Preservatives help to prevent microbial contamination of the composition. They are particularly important when the composition does not comprise a conventional, astringent aluminium containing antiperspirant salt, which is the case in preferred embodiments.

Deodorant actives help counter malodour development on the surface of the human body. They are particularly important when the composition does not comprise a conventional, astringent aluminium containing antiperspirant salt.

The preservative and/or deodorant active is preferably an organic anti-microbial agent. Such agents may be selected from any of those known in the art, provided reasonable skill is used to avoid any incompatibilities. An organic anti-microbial agent is a particularly preferred additional component when the composition used does not include a conventional, astringent aluminium containing antiperspirant salt.

The preservative and/or deodorant active is typically used at a level of from 0.01 to 5% by weight of the composition.

Preferred anti-microbial deodorant agents are those that are more efficacious than simple alcohols such as ethanol. Particularly preferred anti-microbial deodorant agents are soluble in ethanol, meaning that they a solubility in ethanol of at least 10g/L at 20°C. Examples of suitable anti-microbial deodorant agents include niacinamide; quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S. A. Makin and M. R. Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals, 2',4,4'-trichloro,2-hydroxy-diphenyl ether (triclosan), 3,7,11-trimethyldodeca-2,6,10-trienol (farnesol), essential oils such as tea tree oil and thyme oil, climbazole, octapyrox, ketoconazole, zinc pyrithione and mixtures thereof.

Compositions used in accordance with the present invention may optionally include astringent aluminium-containing antiperspirant salts, although in preferred embodiments such salts are excluded.

Astringent aluminium-containing antiperspirant salts include aluminium, zirconium and mixed aluminium/ zirconium salts, including both inorganic salts, salts with organic anions and complexes. Preferred astringent salts include aluminium, zirconium and aluminium/zirconium halides and halohydrate salts, such as chlorohydrates. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in US 3,792,068 (Procter and Gamble Co.). Zinc phenol sulphonate may also be used, preferably at up to 3% by weight of the composition.

When employed, preferred levels of incorporation of conventional astringent aluminium-containing antiperspirant salts are from 0.5% to 60%, particularly from 5% to 40%, and especially from 5% or 10% to 30% or 35% of the composition.

In preferred embodiments of the invention, conventional astringent aluminium-containing antiperspirant salts are not employed.

A preferred optional component of compositions of the invention is a fragrance, typically at a level of from 0.1 to 5% of the total composition. In compositions also comprising water, the fragrance is preferably accompanied by a fragrance solubiliser, typically a nonionic surfactant used a concentration of from 0.1 to 5% of the total composition. In some embodiments, encapsulated fragrances may be employed.

Thickening agents may be employed in compositions of the invention. Such agents increase the viscosity of or solidify the carrier fluid in which the antiperspirant agent is typically suspended or dissolved.

Thickening agents may be selected from any of those known in the art, provided reasonable skill is used to avoid any incompatibilities. A preferred class of thickeners, especially for compositions also comprising water, are hydroxyalkyl celluloses, such as hydroxypropyl cellulose. When employed, thickening agents are typically used at a level of from 0.1 to 40%. When a hydroxyalkyl cellulose thickening agent is employed, this is typically used at a level of from 0.2 to 10% by weight.

In some embodiments, the present invention may involve a stick or soft solid composition. In such compositions, a thickener is an essential additional component and is often described as a gelling agent or structurant.

The thickening agents used in stick compositions according to the invention are preferably selected from fibre-forming non-polymeric gelling agents and waxes, optionally supplemented by particulate silica and/or an oil-soluble polymeric thickening agent.

When employed, the thickening agent often comprises a wax. Thickening waxes typically melt at above 40°C and particularly at between 55 and 95°C. Waxes can include ester waxes, including C12 to C24 linear fatty alcohols, waxes obtained from animals or plants, often after hydrogenation, silicone elastomers and silicone waxes. The thickening agent can comprise a mixture of particulate thickening agents, a mixture of waxes or a mixture of both types of material.

Waxes employed herein as thickening agents are often selected from hydrocarbons, linear fatty alcohols, silicone polymers, esters of fatty acids or mixtures containing such compounds along with a minority (less than 50% w/w and often less than 20% w/w) of other compounds. Naturally occurring waxes are often mixtures of compounds which include a substantial proportion of fatty esters.

Examples of hydrocarbon waxes include paraffin wax, ozakerite, microcrystalline wax and polyethylene wax, the last named desirably having an average molecular weight of from 300 to 600 and advantageously from 350 to 525.

Examples of linear fatty alcohols include those containing from 14 to 40 carbon atoms and often from 16 to 24. Preferred thickening agents of this class are stearyl alcohol and behenyl alcohol, with stearyl alcohol being especially preferred.

Examples of ester waxes include esters of C₁₆-C₂₂ fatty acids with glycerol or ethylene glycol.

Examples of natural waxes include beeswax, wool wax and spermaceti wax of animal origin, and caster wax, jojoba wax, carnauba wax and candelilla wax which are of vegetable origin.

Further waxes employable herein are silicone polymer waxes.

Fibre-forming thickening agents are dissolved in the carrier oil at elevated temperature and on cooling precipitate out to form a network of very thin strands that thicken the carrier oil. Such fibre-forming thickeners include N-acyl amino-acid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

When employed, the thickening agent is typically used at a concentration of from 1.5 to 30%. When a fibre-forming thickening agent is employed, its concentration is typically in the range of from 1.5 to 15%. When a wax is employed, its concentration is usually selected in the range of from 10 to 30%, and particularly from 12 to 24% w/w.

In some embodiments, the present invention may involve an aerosol composition. In such compositions, a volatile propellant is preferred additional component. Preferred volatile propellants are liquefied gases, for example hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Of these especially preferred propellants, isobutane, isobutane/propane, butane/propane and mixtures of propane, isobutane and butane are most preferred.

The liquefied propellant gas is typically the major component of aerosol compositions used in conjunction with the invention, often comprising from 30 to 99% and preferably comprising from 50 to 95% of the total composition.

Other components that may be included in compositions according to the invention including those described in the following paragraphs.

Wash-off agents may be included, often in an amount of up to 10%, to assist in the removal of the formulation from skin or clothing. Such wash-off agents are typically nonionic surfactants such as esters or ethers containing a C₈ to C₂₂ alkyl moiety and a hydrophilic moiety comprising a polyoxyalkylene group (POE or POP).

Skin feel improvers (e.g. talc or finely divided high molecular weight polyethylene), may be included, typically in an amount from 1 up to 10%.

Skin moisturisers, such as glycerol or polyethylene glycol (e.g. mol. wt. 200 to 600) may be included, typically in an amount of up to 5%.

Skin benefit agents, such as allantoin or lipids, may be included, typically in an amount of up to 5%.

Dispensers suitable for use with the present invention comprise containment means and application means for the composition. They also typically comprise means for getting the composition from its containment means to its application means.

In embodiments where the composition is in a liquid form, the containment means is a reservoir for the composition and the application means is preferably a surface from which the composition may be applied to the skin.

Preferred formats for compositions used with the present invention are roll-ons, creams and lotions.

In embodiments where the composition is in a solid or soft solid form, the containment means may be a stick barrel and the application means propels a composition comprising the poly(DMAEMA) clear of the stick barrel and allows it to be rubbed onto skin surface.

In other embodiments, a composition comprising the poly(DMAEMA) is sprayed onto the skin surface. In such embodiments, the containment means is typically a pressurised canister and the application means is a spray nozzle through which the composition is released under pressure on release of a valve.

### Examples

Herein, Examples according to the invention are designated by numbers and Comparative Examples are designated by letters.

The ACH disclosed in these examples is aluminium chlorohydrate, (Al Chlorhydrol 50, from Elementis), a conventional Al-containing antiperspirant active. The active level indicated refers ACH anhydrous solids.

The DMAEMA disclosed in these examples is poly(2-dimethylamino)ethyl methacrylate methyl chloride quaternary salt, from Sigma.

The samples detailed in Table 1 were prepared by the following method. For Comparative Example A and Example 1, an aliquot of model sweat (137.5 µl) was added to an Eppendorf tube, followed by an aliquot of mucin (at 0.8%) in model sweat (312.5 µl). For Comparative Example A, an aliquot of ACH active (at 3%) in water (50 µl) was also added and for Example 1, an aliquot of DMAEMA active (at 3%) in water (50 µl) was also added. For Comparative Example B (control sample), only the model sweat (187.5 µl) and mucin (at 8%) in model sweat ((312.5 µl) were added to the Eppendorf tube. The samples were prepared in triplicate and were each inverted twice to ensure good mixing.

After 2 hours at room temperature, the turbidity of the samples was measured. To do this, the contents of each tube was resuspended with a pipette and samples (100 µl) were placed in a 96 well microtitre plate. The absorbance of each plate at 590 nm was then measured using a plate-reader. The mean results for each sample are shown at the bottom of Table 1.

**Table 1**

| | Example | | |
|---|---|---|---|
| | A | 1 | B |
| Active: | ACH | Poly(DMAEMA) | None |
| Mean turbidity | 0.58 | 0.62 | 0.24 |

For Example 1 and Comparative Example A, the resulting turbidity was significantly greater than that of the control were only mucin was added to model sweat and similar to that obtained with ACH, indicating the potential for significant sweat reduction.

The experimental described above was repeated using various concentrations of poly(DMAEMA) in the 50 µl aliquot added to the mucin and model sweat in the Eppendorf tubes. The results are shown in Table 2.

**Table 2**

| | **Examples** | | | | |
|---|---|---|---|---|---|
| | **A** | **2** | **3** | **4** | **B** |
| Ingredient: | | | | | |
| ACH | 3 | -- | -- | | -- |
| Poly(DMAEMA) | -- | 1.0 | 0.5 | 0.1 | 0 |
| Mean turbidity | 0.572 | 0.664 | 0.413 | 0.239 | 0.227 |

The figures given for ACH and poly(DMAEMA) refer to the concentration of the active in the aliquots added. The concentration in the Eppendorf tube was one tenth of this.

For each of the Examples tested, the resulting turbidity was greater than that of the control were only mucin was added to model sweat, although the difference between the result for Example 4 and Comparative Example B was not significant.

Selected compositions according to the invention are detailed in Tables 3 and 4. These may be prepared by methods known in the art.

**Table 3 - roll-on compositions**

| | **Example** | | | | |
|---|---|---|---|---|---|
| | **5** | **6** | **7** | **8** | **9** |
| Poly(DMAEMA) | 1.0 | 5.0 | 15.0 | 10.0 | 6.5 |
| Steareth-2 | 2.6 | 2.4 | 2.6 | -- | -- |
| Steareth-20 | 0.6 | 0.5 | 0.6 | -- | -- |
| Fragrance | 0.5 | 1.0 | 2.5 | 1.5 | 2.0 |
| Ethanol | -- | -- | -- | 40 | 60 |
| Cellulosic gum | -- | -- | -- | 0.15 | 0.5 |
| Preservative | 0.2 | -- | 0.1 | -- | 0.2 |
| Water | To 100 | To 100 | To 100 | To 100 | To 100 |

**Table 4 - cream and lotion compositions**

| | **Example** | | | | |
|---|---|---|---|---|---|
| | **10** | **11** | **12** | **13** | **11** |
| Poly(DMAEMA) | 5.0 | 5.0 | 15.0 | 10.0 | 6.5 |
| Self-emulsifying wax (Polawax GP200) (structurant) | 5.0 | -- | -- | 10.0 | -- |
| Ethoxylated fatty alcohol (emulsifier) | 4.5 | -- | 2.0 | 4.0 | -- |
| Propylene glycol | 3.0 | -- | -- | -- | |
| Preservative | 0.2 | -- | 0.2 | -- | 0.1 |
| Fragrance | 1.0 | 1.0 | 2.5 | 1.5 | 1.0 |
| Glycerol | -- | 1.0 | -- | 2.0 | -- |
| Isopropyl myristate | -- | -- | 1.7 | -- | -- |
| Ethanol | -- | 70.0 | 55.0 | -- | 75 |
| Deodorant active | -- | 0.2 | 0.3 | -- | -- |
| Water | To 100 | To 100 | To 100 | To 100 | To 100 |

## Claims

1. The non-therapeutic cosmetic use of poly(2-dimethylaminoethyl methacylate) [poly(DMAEMA)] as an antiperspirant agent, wherein the poly(DMAEMA) is a homopolymer.

2. The use according to any one of the preceding claims, wherein the poly(DMAEMA) is of molecular weight 900 Da or greater.

3. The use according to any one of the preceding claims, wherein the poly(DMAEMA) is protonated or quaternized.

## Patentansprüche

1. Nicht-therapeutische kosmetische Verwendung von Poly(2-dimethylaminoethylmethacylat) [Poly(DMAEMA)] als schweißhemmendes Mittel, wobei das Poly(DMAEMA) ein Homopolymer ist.

2. Verwendung nach irgendeinem der vorhergehenden Ansprüche, wobei das Poly(DMAEMA) ein Molekulargewicht von 900 Da oder mehr aufweist.

3. Verwendung nach irgendeinem der vorhergehenden Ansprüche, wobei das Poly(DMAEMA) protoniert oder quaternisiert ist.

## Revendications

1. Utilisation cosmétique non thérapeutique du poly(méthacrylate de 2-diméthylaminoéthyle) [poly(DMAEMA)] comme agent anti-transpirant, dans laquelle le poly(DMAEMA) est un homopolymère.

2. Utilisation selon l'une quelconque des revendications précédentes, dans laquelle le poly(DMAEMA) a un poids moléculaire de 900 Da ou plus.

3. Utilisation selon l'une quelconque des revendications précédentes, dans laquelle le poly(DMAEMA) est protoné ou quaternisé.
